(19) Europäisches Patentamt

European Patent Office

Office européen des brevets

(11) **EP 0 743 862 B1**

(12) **EUROPÄISCHE PATENTSCHRIFT**

(45) Veröffentlichungstag und Bekanntmachung des Hinweises auf die Patenterteilung:
**07.06.2000 Patentblatt 2000/23**

(21) Anmeldenummer: **95909625.6**

(22) Anmeldetag: **08.02.1995**

(51) Int. Cl.[7]: **A61L 29/00**

(86) Internationale Anmeldenummer:
**PCT/DE95/00168**

(87) Internationale Veröffentlichungsnummer:
**WO 95/21635 (17.08.1995 Gazette 1995/35)**

(54) **POLYMERMATERIAL VON MEDIZINISCHEN INSTRUMENTEN UND VERFAHREN ZUR HERSTELLUNG DES POLYMERMATERIALS**

POLYMER MATERIAL FOR MEDICAL INSTRUMENTS AND PROCESS FOR PRODUCING SAID POLYMER MATERIAL

MATERIAU POLYMERE POUR INSTRUMENTS MEDICAUX ET SON PROCEDE DE FABRICATION

(84) Benannte Vertragsstaaten:
**DE DK FR GB IT**

(30) Priorität: **09.02.1994 DE 4404041**

(43) Veröffentlichungstag der Anmeldung:
**27.11.1996 Patentblatt 1996/48**

(73) Patentinhaber: **WILLY RÜSCH AG**
**D-71394 Kernen-Rommelshausen (DE)**

(72) Erfinder:
• **FRITZ, Hans-Gerhard**
**D-73066 Uhingen (DE)**

• **ANDERLIK, Rainer**
**D-69121 Heidelberg (DE)**
• **SINGVOGEL, Armin**
**D-71686 Remseck (DE)**
• **HEIDER, Michael**
**D-71336 Waiblingen-Neustadt (DE)**

(74) Vertreter:
**KOHLER SCHMID + PARTNER**
**Patentanwälte**
**Ruppmannstrasse 27**
**70565 Stuttgart (DE)**

(56) Entgegenhaltungen:
**EP-A- 0 573 884          WO-A-92/18173**

Anmerkung:    Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung geht aus von einem medizinischen Instrument, das aus mindestens einem Schaftabschnitt mit einem Lumen besteht, wobei der oder die Schaftabschnitte aus einem flexiblen Polymermaterial gebildet sind.

Weiterhin betrifft die Erfindung ein Verfahren zur Herstellung eines Polymermaterials.

Derartige medizinische Instrumente, wie etwa Katheter, Schläuche, Trachealtuben usw., werden, wie bekannt, aus einer Vielzahl von Polymerwerkstoffen hergestellt. Dabei kommen sowohl Thermoplaste als auch Elastomere zum Einsatz. Im Bereich der Thermoplaste werden noch zum größten Teil Weich-PVC-Materialien eingesetzt. Aufgrund der niedrigen Erweichungstemperatur von Weich-PVC-Materialien ist bei medizinischen Instrumenten aus diesem Material keine Sterilisierung mit heißem Wasserdampf möglich. Die bekannten medizinischen Instrumente aus Weich-PVC-Material können daher nur einmal steril eingesetzt werden (Einmalinstrument). Dies hat zur Folge, daß bei den Anwendern dieser medizinischen Instrumente große Mengen kontaminierter Instrumente anfallen, die entsorgt werden müssen. Eine Entsorgungsmöglichkeit ist die Verbrennung des kontaminierten PVC-Materials. Dabei entsteht Dioxin, und somit ist diese Art der Entsorgung auch unter höchsten Sicherheitsvorkehrungen umstritten.

Den PVC-Polymeren, die für medizinische Instrumente verwendet werden, werden in der Regel Weichmacher zugesetzt, damit die gewünschte Flexibilität dieser Instrumente erreicht wird. Die Weichmacher werden meist nur physikalisch in das PVC-Polymer eingemischt, so daß die Möglichkeit einer Diffusion dieser Stoffe aus dem PVC-Material in die unmittelbare Umgebung besteht. Sind derartige medizinische Instrumente im menschlichen Körper plaziert, so können die Weichmacher in den Organismus gelangen.

Ein weichmacherfreies, weiches Polymer mit vergleichbarem Eigenschaftsprofil wie Weich-PVC-Material stellen Very Low Density Polyethylene (VLDPE) und die Ultra Low Density Polyethylene (ULDPE) dar, die allerdings ebenso wie das Weich-PVC-Material sehr niedrige Erweichungstemperaturen aufweisen und daher für eine Dampfsterilisierung ungeeignet sind.

Neben den Thermoplastkomponenten, wie sie im medizinischen Bereich eingesetzt werden, werden auch medizinische Instrumente aus Gummiwerkstoffen gefertigt, die als Folge ihrer chemisch vernetzten Struktur den Vorteil der Dampfsterilisierbarkeit aufweisen. Gummiwerkstoffe sind nicht transparent, unterliegen einer aufwendigen Verarbeitungstechnik und sind als Rohstoff - im Vergleich zu Thermoplasten - teuer.

Aufgabe der vorliegenden Erfindung ist es deshalb, ein Polymermaterial für medizinische Instrumente zu entwickeln, das physiologisch unbedenklich, transparent und dampfsterilisierbar ist, sowie die notwendige Flexibilität bei stets geöffnetem Lumen aufweist.

Weiterhin ist es Aufgabe der vorliegenden Erfindung, ein Verfahren für die Herstellung eines derartigen Polymermaterials zur Verfügung zu stellen.

Die Aufgabe bezüglich der Entwicklung des Polymermaterials ist dadurch gelöst, daß das Polymermaterial aus einer ersten Polyethylenkomponente mit niedriger Dichte (VLDPE) und/oder einer zweiten Polyethylenkomponente mit extrem niedriger Dichte (ULDPE) gebildet ist, wobei auf das Polymermaterial ein Organosilan unter Zugabe eines organischen Peroxids aufgepfropft und das Pfropfpolymerisat durch die Lagerung in feuchter Umgebung und/oder in Wasser vernetzt ist.

Bezüglich des Verfahrens zur Herstellung eines derartigen Polymermaterials ist die vorgenannte Aufgabe dadurch gelöst, daß die erste Polyethylenkomponente und/oder die zweite Polyethylenkomponente als rieselfähiges Schüttgut einem Extruder, bevorzugt einem Zweischneckenextruder, gewichtsdosiert zugeführt werden, daß in den Extruder stromabwärts ein Gemisch aus Organosilan und organischem Peroxid und gegebenenfalls einem Katalysator, bevorzugt über eine Membrandosierpumpe mit einem gekühlten Einspritzventil, eingedüst werden, daß am Extruder im Bereich des Produktaustrags ein Vakuum zur Schmelzeentgasung angelegt wird, daß der Extruder mindestens vom Bereich der Zugabe des Organosilans und dem organischen Peroxid bis zum Produktaustrag beheizt ist, und daß aus dem schmelzentgasten, silangepfropften Polymermaterial, unter Feuchteausschluß medizinische Instrumente hergestellt und anschließend unter Feuchteeinwirkung vernetzt werden.

Das erfindungsgemäße Polymermaterial hat den Vorteil, daß es eine erhöhte Knickstabilität gegenüber dem Polymerausgangsmaterial aufweist. Einer unerwünschten Querschnittsverengung des Lumens beim Abbiegen des medizinischen Instruments wird durch das Polymermaterial selbst entgegengewirkt. Weiterhin ist das erfindungsgemäße Polymermaterial weichmacherfrei, weist überraschenderweise die gewünschte Transparenz auf und ist bei der in der Medizin vorgesehenen Temperatur von T = 134°C mehrfach dampfsterilisierbar. Das erfindungsgemäße Polymermaterial nutzt ein Ausgangsmaterial, das kostengünstiger als vergleichbare Gummiprodukte ist, und die Verarbeitung des erfindungsgemäßen Polymermaterials zu medizinischen Instrumenten ist in bekannter Weise und unter Feuchteausschluß möglich.

Die Verarbeitung des erfindungsgemäßen Polymermaterials zu medizinischen Instrumenten kann unmittelbar nach Erhalt der silangepfropften Polyethylenschmelze erfolgen. Die Silanpfropfung und die Schlauchextrusion kann auch in einem Schritt mit einem Extruder durchgeführt werden. Das erfindungsgemäße Polymermaterial kann aber auch nach einer Abkühlung bzw. nach einer weiteren Granulierung weiterverarbeitet werden. Wird das erfindungsgemäße Polymermaterial feuchtedicht in geeigneten Verpackungen (z. B. verschweißte Kunststoffsäcke) gelagert, so kann es noch nach einigen Wochen Lagerzeit gut weiterverarbeitet werden.

Mit dem erfindungsgemäßen Verfahren läßt sich die silangepfropfte Polymerschmelze kostengünstig in einfachen Verfahrensschritten und reproduzierbar herstellen. Über eine bekannte und bewährte Maschinentechnologie werden die Reaktionschemikalien in der ersten und/oder zweiten Polyethylenkomponente homogen verteilt, so daß man ein stippenfreies und gelpartikelfreies Endprodukt erhält.

Werden in bevorzugter Ausgestaltung der Erfindung dem Polymermaterial ein Katalysator bzw. ein Katalysatorgemisch zugefügt, so kann die Vernetzungszeit damit genau festgelegt werden. Ebenfalls verkürzt sich die Bearbeitungszeit, z. B. für das Anschweißen von Komponenten. Als Katalysator eignet sich insbesondere Dibutylzinndilaurat (DBTL) und/oder ein Titanylacetonat. Als Katalysator kann auch ein Gemisch von mehreren geeigneten Chemikalien verwendet werden.

In weiterer Ausgestaltung der Erfindung weist die erste Polyethylenkomponente und/oder die zweite Polyethylenkomponente eine enge Molmassenverteilung auf. Die Molmassenverteilung liegt im Bereich $1,5 \leq \overline{M}_w/\overline{M}_n \leq 3,0$. Dabei stellt $\overline{M}_w$ das gewichtsmittlere und $\overline{M}_n$ das zahlenmittlere Molekulargewicht der Polyethylenkomponente dar. Das Verhältnis von $\overline{M}_w$ zu $\overline{M}_n$, wie es im Patentanspruch 4 formuliert ist, charakterisiert die Breite der Molekulargewichtsverteilung. Vorzugsweise wird erfindungsgemäß eine Polyethylenkomponente oder ein Polyethylenkomponentengemisch ausgewählt, das ein Verhältnis von $\overline{M}_w/\overline{M}_n$ um einen Wert von 2 aufweist. Eine derartige Molmassenverteilung ermöglicht überraschenderweise hohe Gelgehalte im Bereich von $65\,\% \leq C_G \leq 95\,\%$, wie sie mit üblichen VLDPE-Typen nicht erreicht werden können, und hohe Vernetzungsgrade im Endprodukt, wobei hohe Vernetzungsgrade eine Voraussetzung für die Dampfsterilisierbarkeit sind.

In weiterer Ausgestaltung der Erfindung wird ein organisches Peroxid verwendet, dessen Zerfallstemperaturbereich oberhalb der Schmelztemperatur der ersten Polyethylenkomponente und/oder zweiten Polyethylenkomponente liegt. Als organisches Peroxid wird bevorzugt Dicumylperoxid (DCUP), Dibenzoylperoxid (DB) und/oder Dimethylhexanbutylperoxid (DHBP) eingesetzt. Das verwendete Organosilan wird dem oder den Polyethylenkomponenten in Gewichtsbereich von 0,5 bis 5 Gew% und das organische Peroxid im Bereich von 0,02 bis 0,3 Gew% hinzugesetzt.

Als Katalysator werden dem erfindungsgemäßen Polymermaterial Dibutylzinndilaurat (DBTL) im Bereich von 0 bis 0,05 Gew% oder Titanylacetonat im Bereich von 0 bis 0,5 Gew% hinzugefügt.

Mit geringsten Anteilen von Reaktionschemikalien kann aus einen flexiblen Polyethylenmaterial mit niedriger bis extrem niedriger Dichte ein funktionalisiertes Polymermaterial erhalten werden, das sich unter Ausschluß von Feuchte ebenso wie alle Thermoplaste durch Extrusion, Spritzgießen, Blasformen und Schweißen zu medizinischen Formteilen verarbeiten läßt.

In bevorzugter Ausgestaltung des Verfahrens wird die erste Polyethylenkomponente und/oder zweite Polyethylenkomponente vor der Zuführung in den Extruder mit den Reaktionschemikalien bei Umgebungstemperatur in einem Mischgerät gemischt, und das über die Reaktionschemikalien gequollene Schüttgutgemisch wird anschließend dem Reaktionsextruder zugeführt. Über eine derartige Verfahrensweise wird ein sehr homogenes Ausgangsmaterial erreicht, das gegebenenfalls auch in einem Einwellenschneckenextruder zum erfindungsgemäßen Polymermaterial verarbeitet werden kann.

In weiterer Ausgestaltung der Erfindung erfolgt die Wasservernetzung der aus dem erfindungsgemäßen Polymermaterial hergestellten medizinischen Instrumente so lange auf einem Temperaturniveau, das unterhalb der Erweichungstemperatur der Polyethylenausgangsmaterialien liegt, bis durch die eintretende Silanvernetzungsreaktion die Form des Instrumentes stabil ausgebildet ist. Nach dieser Ausbildung bzw. Gestaltsfixierung kann das Temperaturniveau des Vernetzungsbades bis zur vollständigen Vernetzung erhöht werden. Mit dieser Maßnahme läßt sich die Zeit bis zur vollständigen Vernetzung des Polymermaterials verkürzen.

In weiterer Ausgestaltung der Erfindung ist das medizinische Instrument aus einem silangepfropfen Polymermaterial hergestellt, das in einem formgebenden Werkzeug vernetzt ist.

Im bevorzugten Verfahren dazu wird das silangepfropfte Polymermaterial zu Strangextrudaten, wie z.B., Schläuchen, gepresst. Danach wird das Strangextrudat in ein formgebendes Werkzeug eingelegt und das das Strangextrudat beinhaltende Werkzeug wird in ein Wasserbad verbracht.

Die Ultra-low oder Very-low-density-Polyethylene (ULDPE, VLDPE) werden in der Regel in einem Einstufenprozeß, welcher die Silanpfropfung und die Ausformung umfaßt, zu Strangextrudaten gepreßt. Derartige Strangextrudate (Halbzeuge), die am Düsenaustritt einen Gelgehalt < 5% aufweisen, werden anschließend in ein formgebendes Werkzeug gelegt. Sofern aus dem Halbzeug ein medizinisches Instrument hergestellt werden soll, muß vor dem Formvernetzungsschritt noch eine Komplettierung des Halbzeugs zum gewünschten Instrument erfolgen. Anschließend wird das Werkzeug samt Halbzeug bzw. dem komplettierten Instrument in ein Wasserbad verbracht. Abhängig von Pfropfungsrezeptur, Wassertemperatur und Vernetzungszeit wird in dem Halbzeug (Extrudat) durch Vernetzungsreaktion ein dreidimensionales Netzwerk ausgebildet. Die Vernetzung der Makromoleküle erfolgt über Si-O-Si-Brücken. Das ausgebildete Netzwerk fixiert die Geometrie des Extrudates, die während der Vernetzungsphase dem Halbzeug aufgeprägt ist.

Nimmt man nach Abschluß der Vernetzungsreaktion das Formteil aus dem formgebendan Werkzeugteil, so bleibt die Geometrie des Formteils erhalten. Sie ist durch das makromolekulare Netzwerk fest eingeprägt und geht auch im Zuge einer Dampfsterilisation nicht verloren. Dies ist ein erheblicher Vorteil gegenüber medizinischen Instrumenten aus PVC.

Das erfindungsgemäße Polymermaterial und ein Herstellungsverfahren für dieses Material wird in folgendem anhand der in der Zeichnung dargestellten Ausführungsbeispiele näher beschrieben und erläutert. Die der Beschreibung und der Zeichnung zu entnehmenden Merkmale können bei anderen Ausführungsformen der Erfindung einzeln, für sich oder zu mehreren in beliebiger Kombination Anwendung finden. Es zeigt:

Fig. 1    ein Fließschema, wie das erfindungsgemäße Polymermaterial herstellbar ist;

Fig. 2    ein weiteres alternatives Fließschema, das die Herstellung des erfindungsgemäßen Polymermaterials ermöglicht.

Eine erste Polyethylenkomponente 1 bzw. eine zweite Polyethylenkomponente 2 werden entweder für sich alleine oder als Gemisch mit einem Organosilan 3 und einem Peroxid 4 sowie gegebenenfalls auch mit einem Katalysator 5 innig vermischt und einer erhöhten Temperatur im Bereich von $140° \leq T \leq 200°C$ ausgesetzt. Durch eine derartige Wärmebehandlung erhält man ein silangepfropftes Polymermaterial 6, das sich unter Ausschluß von Feuchte wie bekannte Thermoplaste weiterverarbeiten läßt. Beispielsweise kann diese Wärmebehandlung in einem Extruder 7 erfolgen. Die erste Polyethylenkomponente 1 bzw. die zweite Polyethylenkomponente 2 oder ein Polyethylenkomponentengemisch durchlaufen den Extruder 7 in Materialflußrichtung 7'. Im Endbereich des Extruders 7 ist ein Vakuum 8 angelegt, über das die Schmelzeentgasung erfolgt. Die ersten und/oder zweiten Polyethylenkomponenten 1, 2 strömen am Produkteintrag 9 in den Extruder 7 kontinuierlich ein. Das Eintrittsprodukt wird vor dem Eintritt in den Extruder 7 über eine Dosiereinheit 10, beispielsweise eine Differentialdosierwaage, geführt und dort gewichtsdosiert. Das silangepfropfte Polymermaterial 6 verläßt über den Produktaustrag 9' den Extruder 7 und wird anschließend mit bekannten Verfahren granuliert. Der Extruder 7 weist eine Beheizung 11 auf, über die das Polyethylengranulat und das Reaktionsgemisch wärmebehandelt werden.

Die ersten und/oder zweiten Polyethylenkomponenten 1, 2 mit sehr geringen Dichten (z. B. VLDPE, ULDPE) können mit speziellen Polymerisationsverfahren, z. B. unter Verwendung der "constraint geometry catalyst technology" hergestellt werden. Diese Polyethylenkomponenten von denen vorzugsweise Typen mit enger Molmassenverteilung ($1,5 \leq \overline{M}_w/\overline{M}_n \leq 3,0$ vorzugsweise um 2,0) heranzuziehen sind, werden beispielsweise in einem Zweischneckenextruder durch die Zugabe eines Organosilan/Peroxid-Gemisches silangepfropft. Ein gleichsinnig drehender, dichtkämmender Extruder 7 (Zweischneckenextruder) erweist sich für diese Aufgabenstellungen als besonders geeignet, da er eine hohe Misch- und Homogenisierungsleistung aufweist.

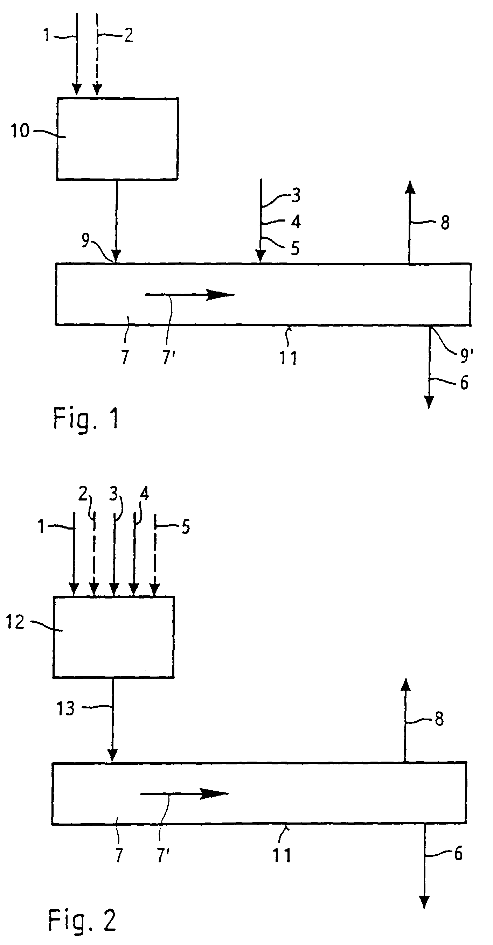

Nach Fig. 1 fördert die Dosiereinheit 10 entweder die erste Polyethylenkomponente 1 bzw. die zweite Polyethylenkomponente 2 oder beispielsweise ein Gemisch aus den vorgenannten Polyethylenkomponenten in Granulatform in den Extruder 7. Nachdem die erste Polyethylenkomponente 1 aufgeschmolzen ist, wird stromabwärts ein Gemisch aus Organosilan 3 und Peroxid 4 gegebenenfalls auch Katalysator 5 mittels einer Membrandosierpumpe und eines gekühlten Einspritzventils in den Extruder 7 eingedüst. Dort wird die Reaktionsflüssigkeit auf reduziertem Temperaturniveau ($90°C \leq T_M \leq 130°C$) innig mit der ersten Polyethylenkomponente 1 vermischt, ehe die Zerfallsreaktion des Peroxids 4 durch eine weitere Temperaturerhöhung längs des Extruders 7 einsetzt. Durch den Zerfall des organischen Peroxids 4 werden in der Polymerkette bzw. den Polymerketten Radikale erzeugt, an die Organosilanmoleküle angekoppelt werden. Geeignete organische Peroxide sind z. B. Dimethylhexanbutylperoxid (DHBP), Dicumylperoxid (DCUP), Dibenzoylperoxid (DB) oder andere Peroxidtypen, bei denen der Zerfall oberhalb der Aufschmelztemperatur der ersten Polyethylenkomponente 1 eintritt. Besonders geeignete Organosilane 3 sind solche, bei denen die Si-Atome mit Alkoxy-Gruppen verknüpft sind. Vorzugsweise werden hier von Vinyltrimethoxysilan (VTMOS) und Vinyltriethoxysilan (VTEOS) eingesetzt. Dem Reaktionsgemisch aus Peroxid 4 und Organosilan 3 können zusätzlich noch Katalysatoren 5 (Vernetzungskatalysatoren), wie etwa Dibutylzinndilaurat (DBTL) oder Titanylacetonat sowie weitere, die Hydrolyse- und Kondensationsreaktion der Organosilane fördernde Katalysatoren, zugefügt werden. Nach der Schmelzeentgasung steht ein silangepfropftes Polymermaterial 6, das einen Ausgangsgelgehalt von 0 % aufweist, zur Verfügung, aus dem mit den üblichen Methoden der Thermoplastverarbeitung medizinische Instrumente hergestellt werden können. Die Verarbeitung des silangepfropften Polymermaterials kann direkt oder erst nach einer Abkühlungs- und Granulierungsstufe erfolgen.

Da beim Silanvernetzungssystem entgegen den üblichen Methoden der Gummiverarbeitung nicht durch eine Temperaturerhöhung, sondern durch die Exposition der medizinischen Instrumente in feuchter Umgebung oder durch Wasserlagerung erfolgt, kann der Beginn der Vernetzung und damit der Zeitraum, der für die Verarbeitung und Konfektionierung des silangepfropften Polymermaterials zur Verfügung steht, durch die Lagerbedingungen festgelegt werden. Genauso kann die Vernetzungsreaktion dadurch beschleunigt werden, daß die aus dem Polymermaterial 6 gefertigten Teile bei erhöhten Temperaturen im Wasser gelagert werden. Dabei ist darauf zu achten, daß die Wasserbadtemperatur nicht die Erweichungstemperatur des Polymermaterials bzw. die Kristallitschmelztemperatur des Polymermaterials ($60° \leq T_e \leq 72°C$) überschreitet. Erst nachdem die Vernetzungsreaktion die Form des medizinischen Instrumentes fixiert hat, können auch über der Erweichungstemperatur des Ausgangsmaterials (unbehandeltes Material) liegende Wasserbadtemperaturen realisiert werden, die die Zeit bis zur vollständigen Vernetzung des Polymermaterials verkürzen.

Die Qualität des silangepfropften Polymermaterials wird außer von der Rezeptierung, d. h. von Organosilan-, Peroxid- und Katalysatoranteilen wesentlich von der Homogenität der Einmischung dieser Reaktionschemikalien in die Polymerschmelze bestimmt. Ist die Verteilung der Reaktionschemikalien inhomogen, so können qualitätsmindernde Stippen und Gelpartikeln im Polymermaterial vorhanden sein.

Fig. 2 zeigt einen Mischer 12, der bevorzugt im Chargenbetrieb arbeitet, in den die erste Polyethylenkomponente 1 bzw. alternativ eine zweite Polyethylenkomponente 2, ein Organosilan 3, ein Peroxid 4 und gegebenenfalls ein Katalysator 5, d. h., eine flüssige Pfropfungsrezeptur, vorgelegt werden. Sowohl die erste Polyethylenkomponente 1 wie auch die zweite Polyethylenkomponente 2 können vorgewärmt sein. In dem Mischer 12 werden diese Komponenten homogen miteinander so lange vermischt, bis die Flüssigkeit vollständig in die in Granulatform vorliegende erste Polyethylenkomponente 1 eindiffundiert ist. Hieraus resultiert eine herausragend gute Vorverteilung der Pfropfungsrezeptur in der Polyethylenmatrix. Lokale Vernetzungsnester und Stippenbildung werden vermieden. Aufgrund der verbesserten Vorverteilung lassen sich die Pfropfungsrezepturen abmagern, um identische Gelgehalte zu realisieren. Erst dann wird ein mit Rekationschemikalien gequollenes Schüttgutgemisch 13 in den Extruder 7 dosiert, wo die Silanpfropfungsreaktion stattfindet. Durch die homogene Vorvermischung kann bei der in Fig. 2 dargestellten Verfahrensvariante anstelle des bewährten Zweischneckenextruders (Zweischneckenkneter) auch ein kostengünstiger Einwellenschneckenextruder mit Mischelementen eingesetzt werden. In Fig. 2 ist der Materialfluß mit 7' gekennzeichnet und eine Schmelzeentgasung findet über das angelegte Vakuum 8 statt. Das silangepfropfte Polymermaterial 6 verläßt den Extruder 7 im Endbereich.

Sowohl die in den Fig. 1 und 2 gezeigten Verfahrensvarianten werden kontinuierlich betrieben.

Versuchsbeispiele:

Die silangepfropften Polymermaterialen I bis IV, deren Zusammensetzung in Tabelle 1 angegeben sind, wurden nach den Herstellungsmöglichkeiten, wie sie in Fig. 1 bzw. Fig. 2 gezeigt sind, hergestellt.

| | Polymermaterial I | Polymermaterial II | Polymermaterial III | Polymermaterial IV |
|---|---|---|---|---|
| Polyethlenkomponente "Exact" Hersteller: Exxon Chemicals | 100 | 100 | 100 | 100 |
| VTEOS (Orangosilan) | 2,5 | - | - | - |
| VTMOS (Organosilan) | - | 2,5 | 2,5 | 2,5 |
| DHBP (Peroxid) | 0,08 | 0,08 | 0,08 | 0,08 |
| DBTL (Katalysator) | | | 0,02 | |
| Titanylacetonat (Katalysator) | | | | 0, 1 |

Nachdem die über einen Trichter in den Extruder dosierte ULDPE-Polyethylenkomponente aufgeschmolzen ist, wird die Mischung aus flüssigen Reaktionschemikalien (Organosilan, Peroxid und gegebenenfalls Katalysator) mittels einer Membrandosierpumpe in den Extruder gepumpt. Dort findet die Silanpfropfungsreaktion statt. Das aus dem Extruder austretende Polymermaterial wird abgekühlt und beispielsweise granuliert. Das aus dem Polymermaterial entstandene Polymergranulat wird unter Feuchteausschluß anschließend mittels eines weiteren Extruders zu beispielsweise Schläuchen verarbeitet. Beim Vergleich der Materialien I und II zeigt sich, daß die Vernetzungsreaktion bei Verwendung von VTMOS deutlich schneller abläuft als mit VTEOS. Werden die silangepfropften Polymermaterialien I und II feuchtedicht in geeigneten Kunststoffsäcken eingeschweißt, so bleiben beide Materialien selbst nach einigen Wochen Lagerzeit noch gut verarbeitbar. In feuchter Umgebung oder im Wasser läuft die Hydrolyse- und Kondensationsreaktion bei VTMOS deutlich schneller als bei VTEOS ab. Zusätzlich kann die Vernetzungsreaktion durch die Zugabe von einem "Masterbatch" im Schlauchextrusionsprozeß erhöht werden. Der "Masterbatch" enthält entweder den Katalysator DBTL oder Titanylacetonat. Befindet sich der Katalysator vom Aufbereitungsprozeß her bereits im Polymermaterial, so verkürzt sich die zur Verfügung stehende Konfektionierungszeit, z. B. für das Anschweißen von Komponenten, ebenso wie die Vernetzungszeit im Wasserbad oder in der feuchten Atomsphäre.

Die vernetzten Schläuche aus den Materialien I bis III sind ebenso transparent wie Schläuche aus unvernetztem ULDPE. Die vernetzten Schläuche aus den Materialien I bis III können problemlos bei einer Temperatur von T = 134°C dampfsterilisiert werden. Ebenso ist die Knickfestigkeit der Schläuche aus den Materialien I bis III gegenüber den unvernetzten Ausgangsmaterialien wesentlich verbessert. Bei Versuch IV (Polymermaterial IV) wurde ein Schüttgutgemisch vor dem Extrusionsprozeß hergestellt und die Polyethylenkomponente wurde so lange mit den flüssigen Reaktionschemikalien gemischt, bis diese vollständig in die Polyethylenkomponente eindiffundiert waren. Danach wurde das Schüttgutgemisch in den Zweiwellenextruder dosiert. Das Polymermaterial IV ist ein silangepfropftes Material mit wenig Stippen und Gelpartikeln (hohe Produktqualität). Das Polymermaterial IV weist eine mit den Polymermaterialien I bis III vergleichbare Transparenz auf. Das Polymermaterial IV ist dampfsterilisierbar und knickfest. Somit weist das Polymermaterial IV die gleichen positiven Produkteigenschaften wie die Polymermaterialien I bis III auf.

Ein Polymermaterial 6 ist aus einer ersten Polyethylenkomponente 1 mit niedriger Dichte (VLDPE) gebildet, das mit einem Organosilan 3 in Verbindung mit organischem Peroxid 4 silangepfropft und durch die Lagerung in feuchter Umgebung und/oder in Wasser vernetzt ist. Das Polymermaterial 6 kann unter Feuchteausschluß vor der Vernetzung zu medizinischen Instrumenten verarbeitet werden. Nach der Vernetzung weisen die aus dem Polymermaterial 6 gefertigten medizinischen Instrumente eine hohe Transparenz auf, sind knickfest und flexibel. Das Polymermaterial 6 wird in einem Extruder, bevorzugt in einem Zweischneckenextruder, hergestellt.

**Patentansprüche**

1. Medizinisches Instrument, das aus mindestens einem Schaftabschnitt mit einem Lumen besteht, wobei der oder die Schaftabschnitte aus einem flexiblen Polymermaterial gebildet sind,
dadurch gekennzeichnet,
daß das Polymermaterial (6) aus einer ersten Polyethylenkomponente (1) mit niedriger Dichte (VLDPE) und/oder einer zweiten Polyethylenkomponente (2) mit extrem niedriger Dichte (ULDPE) gebildet ist, wobei das Polymermaterial (6) mit einem Organosilan (3) in Verbindung mit organischem Peroxid (4) silangepfropft und durch die Lagerung in feuchter Umgebung und/oder in Wasser vernetzt ist.

2. Medizinisches Instrument, nach Anspruch 1, dadurch gekennzeichnet, daß dem Polymermaterial (6) ein Katalysator (5) bzw. ein Katalysatorgemisch hinzugefügt ist.

3. Medizinisches Instrument, nach Anspruch 1 oder 2, dadurch gekennzeichnet, daß die erste Polyethylenkomponente (1) und/oder die zweite Polyethylenkomponente (2) eine enge Molmassenverteilung aufweisen.

4. Medizinisches Instrument, nach Anspruch 3, dadurch gekennzeichnet,
daß die Molmassenverteilung im Bereich von $1{,}5 \leq \overline{M}_w/\overline{M}_n \leq 3{,}0$ liegt.

5. Medizinisches Instrument, nach einem der Ansprüche 1 bis 4, dadurch gekennzeichnet, daß das organische Peroxid (4) einen Zerfallstemperaturbereich oberhalb der Aufschmelztemperatur der ersten Polyethylenkomponente (1) und/oder der zweiten Polyethylenkomponente (2) aufweist, und daß als Peroxid (4) bevorzugt Dicumylperoxid (DCUP), Dibenzoylperoxid (DB) und/oder Dimethylhexanbutylperoxid (DHBP) verwendet wird.

6. Medizinisches Instrument, nach einem der Ansprüche 1 bis 5, dadurch gekennzeichnet, daß das Polymermaterial (6) Organosilan (3) im Bereich von 0,5 bis 5 Gew% und organisches Peroxid (4) im Bereich von 0,02 bis 0,3 Gew% aufweist.

7. Medizinisches Instrument, nach einem der Ansprüche 1 bis 6, dadurch gekennzeichnet, daß das Polymermaterial einen Gelgehalt im Bereich $65\ \% \leq C_G \leq 95\ \%$ aufweist.

8. Medizinisches Instrument, nach einem der Ansprüche 2 bis 7, dadurch gekennzeichnet, daß der Katalysator (5) oder das Katalysatorgemisch ein Dibutylzinndilaurat (DBTL) und/oder ein Titanylacetonat ist oder enthält.

9. Medizinisches Instrument, nach Anspruch 8, dadurch gekennzeichnet, daß Dibutylzinndilaurat im Bereich von 0 bis 0,05 Gew% und/oder Titanylacetonat im Bereich von 0 bis 0,5 Gew% verwendet wird.

**10.** Medizinisches Instrument nach einem der Ansprüche 1 bis 9, dadurch gekennzeichnet, daß das silangepfropfte Polymermaterial (6) in einem formgebenden Werkzeug vernetzt ist.

**11.** Verfahren zur Herstellung eines medizinischen Instruments, das mindestens einen Schaftabschnitt mit einem Lumen aufweist, wobei der oder die Schaftabschnitte aus einem flexiblen Polymermaterial gebildet sind, dadurch gekennzeichnet, daß die erste Polyethylenkomponente (1) und/oder die zweite Polyethylenkomponente (2) als rieselfähiges Schüttgut einem Extruder (7), bevorzugt einem Zweischneckenextruder, gewichtsdosiert zugeführt werden, daß in den Extruder (7) stromabwärts ein Gemisch aus Organosilan (3) und organischem Peroxid (4) und gegebenenfalls einem Katalysator (5), bevorzugt über eine Membrandosierpumpe mit einem gekühlten Einspritzventil, eingedüst werden, daß am Extruder (7) im Bereich des Produktaustrags (9) ein Vakuum (8) zur Schmelzeentgasung angelegt wird, daß der Extruder (7) mindestens vom Bereich der Zugabe des Organosilans (3) und dem organischen Peroxid (4) bis zum Produktaustrag (9') beheizt ist, und daß aus dem schmelzentgasten, silangepfropften Polymermaterial (6) medizinische Instrumente hergestellt und unter Feuchteinwirkung vernetzt werden.

**12.** Verfahren nach Anspruch 11, dadurch gekennzeichnet, daß die erste Polyethylenkomponente (1) und/oder zweite Polyethylenkomponente (2) vor der Zuführung in den Extruder (7) mit den Reaktionschemikalien (3, 4, 5) bei Umgebungstemperatur in einem geschlossenen Mischer (12) gemischt werden, und daß das über die Reaktionschemikalien (3, 4, 5) gequollene Schüttgutgemisch (13) dem Extruder (7) zugeführt wird.

**13.** Verfahren nach Anspruch 11 oder 12, dadurch gekennzeichnet, daß die Wasservernetzung der medizinischen Instrumente so lange auf einem Temperaturniveau erfolgt, das unterhalb der Erweichungstemperatur der ersten und/oder zweiten Polyethylenkomponente (1, 2) liegt, bis durch die eintretende Silanvernetzungsreaktion die Form des medizinischen Instruments stabil ausgebildet ist, und daß dann das Temperaturniveau bis zur vollständigen Vernetzung erhöht wird.

**14.** Verfahren nach einem der Ansprüche 11 bis 13, dadurch gekennzeichnet, daß das silangepfropfte Polymermaterial (6) zu Strangextrudaten, wie z.B., Schläuchen, gepreßt wird, daß das Strangextrudat in ein formgebendes Werkzeug eingelegt wird und daß das das Strangextrudat beinhaltende Werkzeug in ein Wasserbad verbracht wird.

## Claims

**1.** Medical instrument comprising at least one shaft section having a lumen, wherein the shaft section or shaft sections are formed from a flexible polymer material,
characterized in that
the polymer material (6) is formed from a first polyethylene component (1) having low density (VLDPE) and/or a second polyethylene component (2) having extremely low density (ULDPE), wherein the polymer material (6) is silane-grafted with an organic silane (3) in combination with organic peroxide (4) and is cross-linked by storage in a damp environment and/or in water.

**2.** Medical instrument according to claim 1, characterized in that a catalyst (5) or a catalyst mixture is added to the polymer material (6).

**3.** Medical instrument according to claim 1 or 2, characterized in that the first polyethylene component (1) and/or the second polyethylene component (2) have a narrow molar mass distribution.

**4.** Medical instrument according to claim 3, characterized in that
the molar mass distribution is in the range of $1.5 \leq M_w/M_n \leq 3.0$.

**5.** Medical instrument according to one of the claims 1 through 4, characterized in that the organic peroxide (4) has a decomposition temperature region above the melting temperature of the first polyethylene component (1) and/or of the second polyethylene component (2) and dicumylperoxide (DCUP), dibenzoylperoxide (DB) and/or dimethylhexanebutylperoxide (DHBP) are preferentially utilized as peroxide (4).

**6.** Medical instrument according to one of the claims 1 through 5, characterized in that the polymer material (6) contains organic silane (3) in the range between 0.5 to 5 % by weight and organic peroxide (4) in the range between 0.02 to 0.3 % by weight.

**7.** Medical instrument according to one of the claims 1 through 6, characterized in that the polymer material has a gel-

7

content in the range between 65 % $\leq C_G \leq$ 95 %.

8. Medical instrument according to one of the claims 2 through 7, characterized in that the catalyst (5) or the catalyst mixture comprises a dibutyltindilaurate (DBTL) and/or a titanylacetonate.

9. Medical instrument according to claim 8, characterized in that dibutyltindilaurate is utilized in the range between 0 to 0.05 % by weight and/or titanylacetonate in the range between 0 to 0.5 % by weight.

10. Medical instrument according to one of the claims 1 through 9, characterized in that the silane-grafted polymer material (6) is cross-linked in a forming tool.

11. Method for the production of a medical instrument with at least one shaft section having a lumen, wherein the shaft section or sections are formed from a flexible polymer material, characterized in that the first polyethylene component (1) and/or the second polyethylene component (2) are dosed by weight and introduced in the form of a pourable bulk material to an extruder (7), preferentially a double-worm extruder, and a mixture of organic silane (3) and organic peroxide (4) and, if appropriate, a catalyst (5) is injected downstream into the extruder (7), preferentially via a diaphragm dosing pump having a cooled injection valve, and a vacuum (8) is applied to the extruder (7) in the region of product discharge (9') to degas the melt and the extruder (7) is heated at least from the vicinity of the input of the organic silane (3) and the organic peroxide (4) up to the product discharge (9') and medical instruments are produced from the silane-grafted polymer material (6) which was degased in the melt and are cross-linked through the introduction of moisture.

12. Method according to claim 11, characterized in that the first polyethylene component (1) and/or the second polyethylene component (2) are mixed with the reaction chemicals (3, 4, 5) at ambient temperature in a closed mixer (12) prior to introduction into the extruder (7) and the bulk material mixture (13) which has been water-blown by the reaction chemicals (3, 4, 5) is introduced to the extruder (7).

13. Method according to claim 11 or 12, characterized in that the water cross-linkage of the medical instrument is carried out at a temperature level which lies below the softening temperature of the first and/or second polyethylene component (1, 2) for a period of time which is sufficient for the silane cross-linking reactions to stabilize the shape of the medical instrument and the temperature level is subsequently increased to complete cross-linkage.

14. Method according to one of the claims 11 through 13, characterized in that the silane-grafted polymer material (6) is pressed into a moulded extrusion, for example a tube, and the moulded extrusion is introduced into a shaping tool and that tool containing the moulded extrusion is introduced into a water bath.

**Revendications**

1. Instrument médical qui consiste en au moins un segment de tige avec une lumière, où le ou les segments de tige sont constitués par un matériau polymère flexible, caractérisé en ce que le matériau polymère (6) est formé d'un premier composant de polyéthylène (1) à basse densité (VLDPE) et/ou d'un second composant de polyéthylène (2) à densité extrêmement basse (ULDPE), où le matériau polymère (6) est soumis à un greffage de silane avec un organosilane (3) en combinaison avec un peroxyde organique (4) et est réticulé par stockage dans un environnement humide et/ou dans l'eau.

2. Instrument médical selon la revendication 1 caractérisé en ce qu'un catalyseur (5) ou un mélange de catalyseurs est ajouté au matériau polymère (6).

3. Instrument médical selon la revendication 1 ou 2 caractérisé en ce que le premier composant de polyéthylène (1) et/ou le second composant de polyéthylène (2) présentent une distribution des masses moléculaires étroite.

4. Instrument médical selon la revendication 3 caractérisé en ce que la distribution des masses moléculaires est située dans le domaine de $1,5 \leq \overline{M}_p/\overline{M}_n \leq 3,0$.

5. Instrument médical selon l'une des revendications 1 à 4 caractérisé en ce que le peroxyde organique (4) présente un domaine de température de décomposition supérieur à la température de fusion du premier composant de polyéthylène (1) et/ou du second composant de polyéthylène (2) et en ce que, comme peroxyde (4), on utilise de préférence le peroxyde de dicumyle (DCUP), le peroxyde de dibenzoyle (DB) et/ou le peroxyde de diméthylhexanebutyle (DHBP).

**6.** Instrument médical selon l'une des revendications 1 à 5 caractérisé en ce que le matériau polymère (6) comporte l'organosilane (3) dans le domaine de 0,5 à 5% en masse et le peroxyde organique (4) dans le domaine de 0,02 à 0,3% en masse.

**7.** Instrument médical selon l'une des revendications 1 à 6 caractérisé en ce que le matériau polymère présente une teneur en gel dans le domaine $65\% \leq C_G \leq 95\%$.

**8.** Instrument médical selon l'une des revendications 2 à 7 caractérisé en ce que le catalyseur (5) ou le mélange de catalyseurs est ou contient un dilaurate de dibutylétain (DBTL) et/ou un acétonate de titanyle.

**9.** Instrument médical selon la revendication 8 caractérisé en ce que le dilaurate de dibutylétain est utilisé dans le domaine de 0 à 0,05% en masse et/ou l'acétonate de titanyle est utilisé dans le domaine de 0 à 0,5% en masse.

**10.** Instrument médical selon l'une des revendications 1 à 9 caractérisé on ce que le matériau polymère à greffage de silane (6) est réticulé dans un outil de mise on forme.

**11.** Procédé de fabrication d'un instrument médical qui présente au moins un segment de tige avec une lumière où le ou les segments de tige sont formés à partir d'un matériau polymère flexible, caractérisé en ce que le premier composant de polyéthylène (1) et/ou le second composant de polyéthylène (2) sont introduits dosés quant au poids dans une extrudeuse (7), de préférence une extrudeuse à deux vis, sous forme de produit en vrac capable de couler, on ce qu'un mélange d'organosilane (3) et de peroxyde organique (4) et éventuellement d'un catalyseur (5) est introduit dans l'extrudeuse (7) en aval, de préférence par une pompe doseuse à membrane avec une soupape injectrice refroidie, en ce qu'un vide (8) est appliqué à l'extrudeuse (7) dans le domaine de la sortie de produit (9') pour le dégazage de la masse fondue, en ce que l'extrudeuse (7) est chauffée au moins du domaine de l'addition de l'organosilane (3) et du peroxyde organique (4) à la sortie de produit (9') et on ce que des instruments médicaux sont fabriqués à partir du matériau polymère (6) à greffage de silane dégazé à l'état fondu et sont réticulés sous l'action de l'humidité.

**12.** Procédé selon la revendication 11 caractérisé en ce que le premier composant de polyéthylène (1) et/ou le second composant de polyéthylène (2) sont mélangés avec les produits chimiques de réaction (3, 4, 5) à la température ambiante dans un mélangeur fermé (12) avant l'introduction dans l'extrudeuse (7) et en ce que le mélange de produits on vrac (13) gonflé par les produits chimiques de réaction (3, 4, 5) est envoyé à l'extrudeuse (7).

**13.** Procédé selon la revendication 11 ou 12 caractérisé en ce que la réticulation à l'eau des instruments médicaux a lieu à un niveau de température qui est situé sous la température de ramollissement du premier et/ou du second composant de polyéthyléne (1, 2) jusqu'à ce que la forme de l'instrument médical soit constituée de manière stable par la réaction de réticulation au silane qui a lieu, et en ce que le niveau de température est ensuite augmenté jusqu'à la réticulation totale.

**14.** Procédé selon l'une des revendications 11 à 13 caractérisé en ce que le matériau polymère à greffage de silane (6) est extrudé en produits d'extrusion comme des tuyaux souples, par exemple, en ce que le produit d'extrusion est placé dans un outil de mise en forme et en ce que l'outil contenant le produit d'extrusion est disposé dans un bain d'eau.

Fig. 1

Fig. 2